# EUROPEAN PATENT APPLICATION

(11) **EP 4 538 378 A1**
(43) Date of publication of application: **16.04.2025**
(21) Application number: 23823135.1
(22) Date of filing: 13.06.2023
(51) Int. Cl.: C12N 15/64, C12N 15/63, C12N 5/10

(54) **WILD-TYPE-MUTANT ? PROTEIN SWITCHING EXPRESSION SYSTEM CAPABLE OF INCREASING EFFICIENCY OF PREPARING SCREENING-TAG-FREE PLASMID**

(30) Priority: 13.06.2022 CN 202210664659
(71) Applicant: Nanjing GenScript Biotech Co., Ltd., Nanjing, Jiangsu 211100 (CN)
(72) Inventor: ZHOU, Fan, Nanjing, Jiangsu 211100 (CN); ZHANG, Lu, Nanjing, Jiangsu 211100 (CN); LI, Hong, Nanjing, Jiangsu 211100 (CN)
(74) Representative: Synergy IP Group AG
(86) International application number: PCT/CN2023/099902
(87) International publication number: WO 2023/241567

(57) **Abstract**

Provided is a precursor plasmid used for preparing a screening-tag-free plasmid, the precursor plasmid comprising: (1) a conditioned replication initiation site having a plasmid replication initiation capacity that is dependent on regulatory proteins, wherein the conditioned replication initiation site has a first replication initiation state in the presence of a first regulatory protein and a second replication initiation site in the presence of a second regulatory protein, and has a stronger ability to initiate plasmid replication in the second replication initiation state than in the first replication initiation state; 2) a first regulatory protein expression cassette expressing the first regulatory protein; 3) a sequence encoding a repressor protein; 4) a screened tag gene; 5) a target gene, or a cloning site for inserting the target gene; and 6) paired recombination sites. Also provided are a host cell suitable for the recombination of the precursor plasmid and the production of a screening-tag-free plasmid, and a method for large-scale production of the screening-tag-free plasmid.

## Description

### Cross Reference to Related Application

The present application claims the right of priority for Chinese Patent Application No. CN202210664659.1 filed on June 13, 2022, which is incorporated herein by reference in its entirety.

### Technical Field

The present invention relates to a method and a kit for preparing a screening-tag-free plasmid, especially a method and a kit for the large-scale preparation of a screened-tag-gene-free plasmid by using wild-type and mutant II proteins to control plasmid replication in an orderly manner.

### Background Art

In recent years, gene and cell therapy technologies are gradually becoming new important approaches to treating human diseases. Safe and efficient DNA delivery vectors enable gene and cell therapy to play a greater role in the prevention and treatment of human diseases in the future. Over the past two decades, non-viral delivery systems based on plasmid vectors and viral packaging/delivery systems based on plasmids have received widespread attention in areas such as gene defect repair, disease treatment and prevention. Improving the safety, stability and yield of plasmids and reducing cytotoxicity have been a major focus of research for plasmids in the field of gene and cell therapy.

Plasmid DNA molecules used in gene therapy usually have a modular structure comprising a eukaryotic transcription unit and a prokaryotic replication unit. In addition to the sequences required for DNA replication, conventional plasmids usually carry at least one antibiotic drug resistance gene for ease of positive cloning screening and stable inheritance during the cloning process. Common screening tags include ampicillin, kanamycin, chloramphenicol, neomycin, tetracycline, etc. (Davies, J and Smith, D I (1978). Plasmid-Determined Resistance to Antimicrobial Agents. Annual Review of Microbiology, 32(1), 469-508). However, when applied to gene therapy, plasmid DNA may be taken up by bacteria present on the respiratory or digestive tract surface, and antibiotic resistant genes carried on the plasmid may cause side effects such as antibiotic resistance in patients, thus contributing to the development of antibiotic-resistance-tag-free plasmids.

The more widely used antibiotic-resistance-tag-free plasmids, including Minicircles and RNA-OUT screening-based plasmids, have the antibiotic screening tags deleted, and as a result, a large reduction in the proportion of sequences of bacterial origin and a reduction in the toxicity of a plasmid vector to a host cell are achieved. With the widespread use of gene therapy, there is an urgent need to develop a method for producing high-yield, high-purity antibiotic-resistance-tag-free plasmids.

### Summary of the Invention

In one aspect, provided herein is a precursor plasmid comprising:
1) a conditioned replication initiation site having a plasmid replication initiation capacity that is dependent on regulatory proteins, wherein the conditioned replication initiation site has a first replication initiation state in the presence of a first regulatory protein and a second replication initiation state in the presence of a second regulatory protein, and has a stronger ability to initiate plasmid replication in the second replication initiation state than in the first replication initiation state;
2) a first regulatory protein expression cassette expressing the first regulatory protein;
3) a sequence encoding a repressor protein;
4) a screened tag gene;
5) a target gene, or a cloning site for inserting the target gene; and
6) paired recombination sites,

wherein the paired recombination sites enable the self-recombination of the precursor plasmid in the presence of a recombinase to form a subplasmid and a circular double-stranded DNA,
wherein the subplasmid comprises the conditioned replication initiation site and the cloning site, or comprises the conditioned replication initiation site and the target gene, and the circular double-stranded DNA comprises the screened tag gene, the first regulatory protein expression cassette, and the sequence encoding a repressor protein.

In some embodiments, the presence of the repressor protein enables the inhibition of expression of the second regulatory protein in a host cell expressing the second regulatory protein upon introduction of the precursor plasmid into the host cell.

In some embodiments, the host cell comprises a second regulatory protein expression cassette expressing a second regulatory protein, wherein the second regulatory protein expression cassette comprises an expression regulatory sequence, and when bound to the expression regulatory sequence, the repressor protein inhibits the expression of the second regulatory protein.

In some embodiments, the sequence encoding a repressor protein is located in the first regulatory protein expression cassette such that the repressor protein is expressed in tandem with the first regulatory protein.

In some embodiments, the conditioned replication initiation site is the *ori_{R6Kγ}* replication initiation site.

In some embodiments, the first regulatory protein is a wild-type II protein and the second regulatory protein is a mutant of the wild-type II protein.

In some embodiments, the second regulatory protein is a product of *pir-116* coding gene.

In some embodiments, the *pir-116* coding gene comprises the sequence as shown in SEQ ID NO: 2.

In some embodiments, the conditioned replication initiation site comprises the sequence as shown in SEQ ID NO: 8, or a nucleotide sequence having at least 80% identity to the sequence as shown in SEQ ID NO: 8 and capable of exerting the replication initiation function.

In some embodiments, the sequences of the paired recombination sites are direct sequences.

In some embodiments, the paired recombination sites are direct loxP sequences, and the recombinase is a Cre recombinase; the paired recombination sites are direct FRT sequences, and the recombinase is a Flp recombinase; or the paired recombination sites are direct attB/attP sequences, and the recombinase is a PhiC31 recombinase.

In some embodiments, the paired recombination sites are direct lox71 and lox66 sequences.

In some embodiments, the repressor protein is LacI protein.

In some embodiments, the expression regulatory sequence comprises a *lacO* operator sequence.

In some embodiments, the *lacO* operator sequence comprises the sequence as shown in SEQ ID NO: 1.

In some embodiments, the promoter of the first regulatory protein expression cassette is a *P_{lacl}^{q}* promoter. Preferably, the *P_{lacl}^{q}* promoter comprises the sequence as shown in SEQ ID NO: 20.

In some embodiments, the screened tag gene is an antibiotic resistant gene.

In another aspect, provided herein is a host cell comprising:
1) a recombinase expression cassette expressing a recombinase; and
2) a second regulatory protein expression cassette expressing a second regulatory protein,
wherein the second regulatory protein expression cassette comprises an expression regulatory sequence, and when bound to the expression regulatory sequence, the repressor protein inhibits the expression of the second regulatory protein.

In some embodiments, the recombinase is a Cre recombinase, a Flp recombinase, or a PhiC31 recombinase.

In some embodiments, the recombinase expression cassette is an inducible recombinase expression cassette, preferably an arabinose-inducible expression cassette.

In some embodiments, the second regulatory protein is a product of *pir-116* coding gene. Preferably, the *pir-116* coding gene comprises the sequence as shown in SEQ ID NO: 2.

In some embodiments, the repressor protein is LacI protein.

In some embodiments, the expression regulatory sequence comprises a *lacO* operator sequence. Preferably, the *lacO* operator sequence comprises the sequence as shown in SEQ ID NO: 1.

In some embodiments, the recombinase expression cassette and/or the second regulatory protein expression cassette are integrated in the genome of the host cell.

In some embodiments, the host cell is *Escherichia coli.*

In another aspect, provided herein is a method for preparing a screened-tag-gene-free plasmid, comprising:
I) preparing a precursor plasmid comprising
   1) a conditioned replication initiation site having a plasmid replication initiation capacity that is dependent on regulatory proteins, wherein the conditioned replication initiation site has a first replication initiation state in the presence of a first regulatory protein and a second replication initiation state in the presence of a second regulatory protein, and has a stronger ability to initiate plasmid replication in the second replication initiation state than in the first replication initiation state;
   2) a first regulatory protein expression cassette expressing the first regulatory protein;
   3) a sequence encoding a repressor protein;
   4) a screened tag gene;
   5) a target gene, or a cloning site for inserting the target gene; and
   6) paired recombination sites,
   wherein the paired recombination sites enable the self-recombination of the precursor plasmid in the presence of a recombinase to form a subplasmid and a circular double-stranded DNA, wherein the subplasmid comprises the conditioned replication initiation site and the cloning site, or comprises the conditioned replication initiation site and the target gene, and the circular double-stranded DNA comprises the screened tag gene, the first regulatory protein expression cassette, and the sequence encoding a repressor protein;
II) introducing the precursor plasmid into a host cell, wherein the host cell comprises:
   1) a recombinase expression cassette expressing the recombinase; and
   2) a second regulatory protein expression cassette expressing the second regulatory protein,
   wherein the second regulatory protein expression cassette comprises an expression regulatory sequence, and when bound to the expression regulatory sequence, the repressor protein inhibits the expression of the second regulatory protein;
III) screening for the host cell expressing the screened tag gene;
IV) culturing the host cell screened in step III), allowing the expression of the recombinase in the host cell, continuing to culture the host cell and screening for the host cell not expressing the screened tag gene; and
V) culturing the host cell screened in step IV) and extracting the plasmid.

In some embodiments, the presence of the repressor protein enables the inhibition of expression of the second regulatory protein in a host cell upon introduction of the precursor plasmid into the host cell.

In some embodiments, the sequence encoding a repressor protein is located in the first regulatory protein expression cassette such that the repressor protein is expressed in tandem with the first regulatory protein.

In some embodiments, the conditioned replication initiation site is the *ori_{R6Kγ}* replication initiation site.

In some embodiments, the first regulatory protein is a wild-type II protein and the second regulatory protein is a mutant of the wild-type II protein.

In some embodiments, the second regulatory protein is a product of *pir-116* coding gene.

In some embodiments, the sequences of the paired recombination sites are direct sequences.

In some embodiments, the paired recombination sites are direct loxP sequences, and the recombinase is a Cre recombinase; the paired recombination sites are direct FRT sequences, and the recombinase is a Flp recombinase; or the paired recombination sites are direct attB/attP sequences, and the recombinase is a PhiC31 recombinase.

In some embodiments, the paired recombination sites are direct lox71 and lox66 sequences.

In some embodiments, the repressor protein is LacI protein and the expression regulatory sequence comprises a *lacO* operator sequence.

In some embodiments, the screened tag gene is an antibiotic resistant gene.

In some embodiments, the recombinase expression cassette is an inducible recombinase expression cassette, preferably an arabinose-inducible expression cassette.

In some embodiments, the recombinase expression cassette and/or the second regulatory protein expression cassette are integrated in the genome of the host cell.

In some embodiments, the host cell is *Escherichia coli.*

In some embodiments, the allowing the expression of the recombinase in the host cell in step IV) is achieved by adding to the host cell an inducer corresponding to the inducible recombinase expression cassette.

In another aspect, provided herein is the use of the above precursor plasmid or host cell in the preparation of a screened-tag-gene-free plasmid.

In another aspect, provided herein is a kit for the preparation of a screened-tag-gene-free plasmid, comprising the above precursor plasmid and/or host cell.

In some embodiments, the subplasmid is capable of replicating in a host cell, wherein the host cell may be *Escherichia coli,* e.g., the host cell may be *Escherichia coli* JM108, TOP10, DH5α, GT115, pir1, pir2, etc., and other engineered strains derived from related background strains. The subplasmid is fermented in a host cell and obtained by plasmid extraction.

Using the precursor plasmid and host cell provided herein and/or the above method, the large-scale production of subplasmids (screening-tag-free subplasmids) can be achieved to meet production requirements.

### Brief Description of the Drawings

FIG. 1 is a schematic diagram of the structure of the precursor plasmid provided herein, wherein the R6K γ ori represents replication initiation site; Lox71 and Lox66 represent recombination sites; Redundant backbone including Kan^{R} Gene represents a backbone sequence containing an antibiotic resistant gene; MSC represents a multiple cloning site; pirWT-lacI represents a wild-type II protein coding gene - operon repressor protein tandem expression cassette.
FIG. 2 is an electrophoretogram showing amplification results of the pKD46 plasmid fragment, Lac and *pir116* fragments.
FIG. 3 shows the sanger sequencing results of the constructed pKD46-Lac-pir116 plasmid.
FIG. 4 is an electrophoretogram showing the PCR validation results for engineered strain *JM108-cre-pir116.*
FIG. 5 shows the sanger sequencing results of the engineered bacterial gene JM108-*cre-pir116* prepared herein.
FIG. 6 is an electrophoretogram showing amplification results of the pMF5 fragment, *lacI* fragment and *pirWT-loxp* empty plasmid fragment.
FIG. 7 shows the sanger sequencing results of the empty plasmid pMF5*-lacI-pirWT-loxp* constructed herein.
FIG. 8 is an electrophoretogram showing amplification results of fragments of different sequence lengths.
FIG. 9 is an electrophoretogram showing the results of validation 1 h after induced recombination.
FIG. 10 shows the results of the resistance plate screening for resistance-lost clones.
FIG. 11 is an electrophoretogram showing the results of validation of the purified plasmid for the resistance plate.
FIG. 12 is an electrophoretogram showing the results of validation of large-scale plasmid extraction.

### Detailed Description of Embodiments

Unless otherwise indicated, all technical and scientific terms used herein have the meanings as commonly understood by one of ordinary skill in the art. For the purpose of facilitating the understanding of the technical solutions provided herein, some technical terms are briefly described below.

"Plasmid" and "plasmid vector" are used interchangeably herein to refer to a circular DNA molecule that carries a replication initiation site (or DNA replication element) and thereby has the capacity to replicate autonomously in a host cell. Plasmids can be native or engineered. A plasmid may comprise a screened tag gene, such as an antibiotic resistant gene, such that a host cell containing the plasmid can grow under a specific culture condition, whereas a host cell not containing the plasmid cannot grow normally under that specific culture condition. For example, when a host cell (such as *Escherichia coli*) contains a plasmid with a tetracycline resistance gene, it can grow in a medium containing tetracycline, and when the host cell does not contain or loses such a plasmid, it can no longer grow in a medium containing tetracycline or its growth is inhibited therein. Thus, the screening function is accomplished by using the screened tag gene to enable the technician to know which host cells contain the desired plasmid. Plasmids may also include, or have been engineered to include cloning sites to facilitate the insertion of a target gene. After inserted into the plasmid by a cloning site, the target gene can be replicated with the plasmid to achieve the amplification of the target gene; alternatively, when constructed as an expression plasmid, the plasmid can be used for expression of a target gene in a host cell. The cloning site can be a single cloning site or a multiple cloning site. A multiple cloning site is usually preferred for ease of experimental operation. The multiple cloning site used herein refers to a DNA segment containing multiple sites recognized by restriction endonucleases or other endonucleases (such as homing endonucleases). The restriction endonuclease can be, for example, *Ahd I, AclI, HindIII, SspI, MluCI, Tsp509I, PciI, AgeI, BspMI, BfuAI, SexAI, MluI, BceAI, Nde I or EcoR I.*

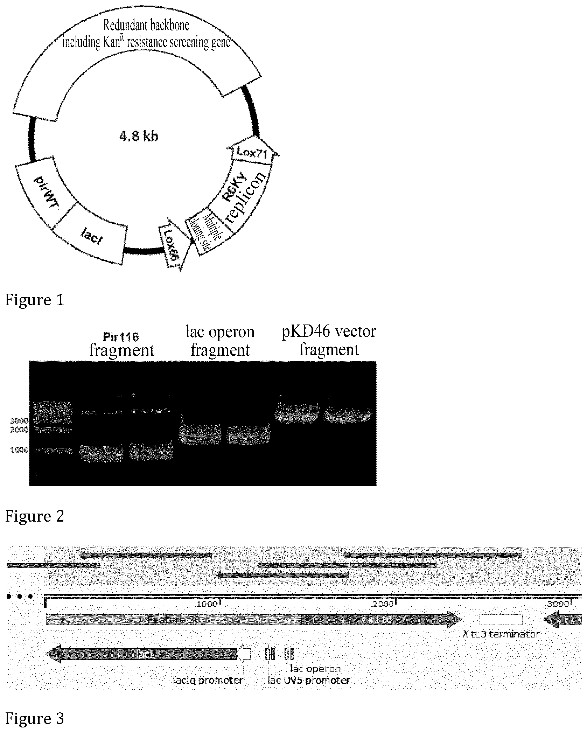

A "precursor plasmid" is used herein to refer to the parent plasmid used to produce a target plasmid/subplasmid (e.g., a screened-tag-gene-free plasmid). In order to generate a screened-tag-gene-free plasmid, a screened tag gene is usually placed between the paired recombination sites (see FIG. 1). Thus, when a recombination reaction occurs, one precursor plasmid molecule forms two circular DNA molecules, wherein one of the circular DNA molecules contains a replication initiation site and a target gene or cloning site, without a screened tag gene; correspondingly, the other circular DNA molecule contains the screened tag gene, without the replication initiation site and cloning site. The former is also referred to herein as a subplasmid or a target plasmid, i.e., a screened-tag-gene-free plasmid. For the purposes of the present invention, when reference is made to a cloning site, it encompasses the cloning site itself, or a cloning site into which a target gene has been inserted. Alternatively, a precursor plasmid (or "platform plasmid") that does not contain a target gene at the cloning site may be considered to be the parent plasmid of a precursor plasmid containing a target gene at the cloning site, all of which are included within the meaning of "precursor plasmid" herein.

A "subplasmid" is also referred to herein as a "miniplasmid" and a "target plasmid", which refers to a sequence containing a replication initiation site and a cloning site and/or a target gene produced by a precursor plasmid containing a recombination site (such as direct LoxP sequences) experiencing a recombination reaction in the presence of a recombinase (e.g. a Cre recombinase). In some implementations herein, the subplasmid comprises a replication initiation site and a cloning site and/or a target gene sequence, but does not comprise a screened tag gene.

A "recombination site" herein refers to a nucleotide sequence that is specifically recognized by the corresponding recombinase. When the precursor plasmid contains paired direct recombination sites, the recombination reaction takes place between the recombination sites within the precursor plasmid under the action of the corresponding recombinase, resulting in the production of one copy of subplasmid and one copy of circular DNA. In some embodiments, the recombination sites are loxP sequences and the corresponding recombinase is a Cre recombinase; in other embodiments, the recombination sites are FRT sequences and the corresponding recombinase is a Flp recombinase. Those skilled in the art will appreciate that recombination sites employed in a precursor plasmid are feasible as long as sequences (comprising the screened tag gene) between the sequences are knocked out of the precursor plasmid at the time of the recombination reaction and the remaining sequences form a subplasmid. These recombination sites are therefore not limited to the specific sequences mentioned herein, but may also be variants thereof, or other recombination sites. For example, the recombination site LoxP mutants can be lox75, lox44, lox76, lox43, lox72, lox78, lox65, lox511, lox5171 or lox2272. The recombination site FRT may be wild-type or mutant, such as FRT3 and FRT5. In a specific embodiment herein, the recombination site loxP represents lox71 and lox66 sequences with nucleotide sequences as shown in SEQ ID NOs: 10 and 11, respectively.

A "host cell" herein refers to a cell, including prokaryotic and eukaryotic cells, e.g., bacteria (such as *Escherichia coli),* fungi (yeast), insect cells and mammalian cells, in which a plasmid can be maintained and/or replicated. In the method provided herein for preparing a screened-tag-gene-free plasmid, the host cell provides the necessary ingredients (e.g., various enzymes and nucleotide monomer molecules) for replication of a plasmid therein, as well as the recombinase required for the recombination reaction. The expression of a recombinase in a host cell is preferably controlled or inducible. In some embodiments, a gene encoding the recombinase is integrated into the genome of a host cell. In other embodiments, the gene encoding the recombinase is placed in an additional expression vector, which expression vector may be introduced into a host cell concurrently with, before or after the precursor plasmid. In either case, it is preferable to place the recombinase gene under the control of an inducible promoter so that the technician can control the time at which the recombination reaction occurs.

A "conditioned replication initiation site" is used herein to refer to such a replication initiation site that is dependent on the presence of other factors to initiate plasmid replication. In one example, the conditioned replication initiation site is *ori_{R6Kγ,}* which requires the presence of a II protein to initiate plasmid replication.

The "replication initiation state" herein refers to the ability of the replication initiation site to initiate plasmid replication. Different replication initiation states can be confirmed by detecting the number of copies or the change thereof of the plasmid containing the replication initiation site in the same host cell. A replication initiation site contained in a precursor plasmid has at least two replication initiation states: a first replication initiation state and a second replication initiation state, wherein the replication initiation site has a stronger ability to initiate plasmid replication in the second replication initiation state than in the first replication initiation state. For example, when the replication initiation site in the plasmid is in the first replication state, the plasmid has no more than 15 copies in the host cell, for example in the case of a single copy plasmid; however, when the replication initiation site is in the second replication state, the plasmid has a significantly higher copy number in the host cell, for example, 50 or more (e.g., 75, 100, 150, 200, or 250). In some embodiments, the replication initiation site may be controlled or maintained in a first replication initiation state or a second replication initiation state according to the presence of a first regulatory protein or a second regulatory protein in the host cell, respectively. For example, for the replication initiation site *ori_{R6Kγ,}* the first regulatory protein can be a wild-type II protein, and the second regulatory protein can be a mutant of the wild-type II protein. In a specific example, the mutant of the wild-**type** II **protein** is the mutant pir-116 (with proline replaced with leucine at position 106 relative to the wild type). In other examples, mutants of the wild-type II protein are mutants different from pir-116, such as pir-42, pir-108 and pir-113. Miron, A et al. have carried out a detailed study of the mechanism of action of different mutants of II protein on the replication initiation site *ori_{R6Kγ,}* and expounded the methods of obtaining these mutants, see, for example, Miron, A et al., EMBO J., 1992(11): 1205-1216, and Miron, A et al., PNAS USA,1994(91): 6438-6442, which are hereby incorporated herein by reference in their entireties.

In the plasmid recombination and resistance-tag-free screening stages prior to a large-scale plasmid preparation, it is desirable to have a lower copy number (e.g. single copy) of the plasmid in a host cell, so that the replication initiation site on the plasmid is maintained in a first replication initiation state (i.e. the first regulatory protein is present in the host cell), whereas in the large-scale plasmid preparation stage, it is desirable to have a higher copy number of the precursor plasmid in the host cell, so that the replication initiation site on the plasmid is in a second replication initiation state (i.e., the second regulatory protein is present in the host cell).

*Ori_{R6Kγ}* is a conditioned replication initiation site which is dependent on the II protein encoded and expressed by the *pir* gene to initiate replication. In order to maintain the conditioned replication of a plasmid containing *ori_{R6Kγ}* but lacking *pir* gene, II protein is expressed by the *pir* gene located on a compatible plasmid in a trans-regulation mode, or by the *pir* gene inserted into the host chromosome through homologous recombination. Usually, some mutations in the *pir* gene can change the copy number of *R6K* plasmid, and the mutant *pir-116* has been determined to increase the copy number of plasmid. One *ori_{R6K γ}* plasmid has 15 copies per cell or even less in the wild-type *pir+* host, which is dependent on the size of the *ori_{R6Kγ}* plasmid; the mutant *pir-116* is obtained by replacing proline with leucine at the position 106 starting from the start codon of *pir* gene, and the *ori_{R6Kγ}* plasmid has 250 copies per cell in the mutant. *Ori_{R6Kγ}* can avoid the uncontrolled over-expression of a delivery plasmid *in vivo* to a great extent while minimizing the DNA delivery vector, so it is a preferred target of a DNA delivery vector.

In the international application for patent of the preliminary research PCT/CN2021/133141 "PLASMID SYSTEM WITHOUT SELECTABLE MARKERS AND PRODUCTION METHOD THEREOF" filed by inventor(s), a safe and efficient DNA delivery vector is developed and produced, which carries only one DNA replication element such as *ori_{puc}* or *ori_{R6Kγ},* compared to Minicircles and RNA-OUT screening-based plasmid delivery vectors in the prior art, and makes it possible to reduce the proportion of sequences of bacterial origin, to enable plasmid self-replication, and to meet the needs of simple production process and scalable production, with *ori_{R6Kγ}* minimizing the DNA delivery vector.

In order to further optimize the screening-tag-free plasmid system and production method, and to achieve the goal of increasing the yield of the plasmid while reducing the proportion of sequences of bacterial origin in the product plasmid, the present application provides a wild-type-mutant II protein switching expression system capable of increasing efficiency of preparing a screening-tag-free plasmid. In the system, *pir⁺*(wild-type) and *pir-116* genes are located on the precursor plasmid and the *Escherichia coli* chromosome, respectively, and are expressed in sequence during gene expression, without the need for an additional induction process. In the small volume culture stage, the expression of the *pir-*116 gene is repressed, with replication carried out in low copies to control the precursor plasmid in a haploid form to complete the recombination and the screening-tag-free plasmid screening; and in the large-scale preparation stage, the *pir⁺* gene is lost after recombination, and the *pir-116* gene initiates expression, with replication carried out in high copies to increase plasmid yield, thereby completing the large-scale production of screening-tag-free plasmids. The recombination system succeeds in reducing the proportion of sequences of bacterial origin in the product plasmid while increasing the plasmid yield.

In some embodiments, a method for preparing a screening-tag-free plasmid provided herein comprises:
(1) further constructing a mutant II protein manipulation expression system on the basis of the constructed engineered *Escherichia coli* strain JM108-*araBAD-cre,* wherein the mutant II protein is encoded by the *pir-116* gene;
(2) constructing a precursor plasmid of an *R6K* replication system that can be recombined into a loop, wherein the precursor plasmid contains a target gene, an *R6K* replication element, a pair of specific direct recombination sites, antibiotic resistant genes, the *lacI^{q}* promoter and the *lacI* gene and the wild-type II protein coding gene *pirWT* expressed in tandem by the *lacI^{q}* promoter;
(3) transforming the above precursor plasmid and screening for positive clones in the engineered strain JM108-*araBAD-cre-pir116*;
(4) culturing cells, with the precursor plasmid replicating in low copies under the action of the II protein expressed by the *pirWT* gene; inducing the expression of recombinase to make the precursor plasmid self-recombine, with 1 molecule of the precursor plasmid forming 1 molecule of resistance-screening-tag-free miniplasmid containing a target gene and an R6K replication element and 1 molecule of circular double-stranded DNA containing plasmid backbone sequences such as an antibiotic resistant gene, *lacI* gene and *pirWT* gene;
(5) performing isolation and purification and screening for an antibiotic-resistance-free monoclonal strain, with the antibiotic-resistance-screening-tag-free plasmid replicating in high copies under the action of the II protein encoded and expressed by the *pir-116* gene; and
(6) culturing cells and extracting plasmids.

In a more specific embodiment, a method for preparing a screening-tag-free plasmid provided herein comprises:
(1) constructing a mutant II protein manipulation expression system, wherein the mutant II protein manipulation expression system is integrated into the genome of an engineered *Escherichia coli* that has already contained a site-specific recombinase-inducible expression system for expression; the composition of the mutant II protein manipulation expression system comprises: an expression regulatory region of a *Lac* operon, including a promoter (such as the promoter P_{lacUV5-tac}) and an operator sequence (such as *a lacO operator sequence),* mutant II protein coding gene *pir-116,* and a transcription terminator;
(2) constructing a precursor plasmid that can be recombined into a loop, wherein the precursor plasmid comprises: DNA replication element *ori_{R6Kγ},* a target gene, a pair of specific direct recombination sites, a screened tag gene (such as an antibiotic resistant gene), the *Lac* operon repressor protein coding gene *lacI* and the wild-type II protein coding gene *pirWT* expressed in tandem (the tandem genes have their expression initiated by *P_{lacf}q*), with the target gene not containing specific recombination sites of the same type as the plasmid backbone; in addition to the target gene sequence and the DNA replication element, plasmid backbone sequences such as the screened tag gene and the tandem expression genes are located within a pair of specific direct recombination sites; after induction of recombination, a precursor plasmid can be reconstituted under the action of a site-specific recombinase and split to form 2 circular double-stranded DNAs, one of which contains plasmid backbone sequences such as the screened tag gene and the tandem expression genes, does not have the ability to replicate, and can be gradually lost during cell expansion; the other of which is a miniplasmid containing only DNA replication elements and the target gene sequence, which is continuously amplified as the cell proliferates;
(3) transforming the precursor plasmid and screening for positive clones in the above genetically engineered strain; the above genetically modified strain is prepared into a competent cell, the precursor plasmid is transformed, and positive clones are screened using a selective medium corresponding to the screened tag gene on the precursor plasmid;
(4) culturing cells and inducing recombination, wherein the transformed positive clones in (3) are picked and the enriched bacteria are cultured overnight in a liquid medium containing antibiotics; the *pirWT* gene carried by the precursor plasmid encodes and expresses a II protein, the R6K replicon is regulated to complete plasmid replication, meanwhile, a *lacI* gene expression product binds to the *Lac* operon operator gene of the mutant II protein expression system on the *Escherichia coli* genome to repress the expression of the mutant II protein by the *pir-116* gene; cells are washed with a liquid medium without selective pressure, the bacteria are resuspended in a medium without selective pressure containing an inducer, and the expression of a recombinase is induced so that recombination occurs between the two recombination sites carried by the plasmid itself, resulting in the deletion of plasmid backbone sequences such as the antibiotic resistant gene and the tandem expression genes between recombination sites and formation of a screening-tag-free miniplasmid; as recombination occurs, the tandem expression genes of the precursor plasmid are gradually lost during cell expansion, and the repression of *pir-116* gene coding and expression by the *lacI* gene expression products is gradually weakened, so the coding gene of II protein is gradually switched from *pirWT* of the precursor plasmid to *pir-116* of Escherichia coli genome;
(5) performing isolation and purification and screening for an antibiotic-resistance-free monoclonal strain, wherein the bacterial liquid completely recombined in (4) is streaked on a plate without selective pressure to separate a single colony; a plurality of single colonies are picked and spotted on a resistance plate, and a resistance-free LB liquid medium is correspondingly inoculated with these colonies, and cultured overnight; colonies that can not grow on the resistance plate but can correspondingly grow in the resistance-free LB liquid medium are selected for culturing to obtain strains containing only screening-tag-free plasmids; and
(6) culturing strains obtained in (5) in a resistance-free medium, and extracting the plasmid to obtain the final product of the screening-tag-free miniplasmid.

Provided herein are antibiotic-gene-free plasmids and production methods therefor, wherein the plasmids can be used as DNA delivery vectors or viral packaging plasmid vectors for gene and cell therapy applications to improve plasmid safety and stability, and reduce cytotoxicity.

Beneficial technical effects of the present invention include, but are not limited to:
1. plasmids without antibiotic screening tags and no excess prokaryotic DNA elements other than replication sites;
2. no antibiotic drug addition during plasmid production;
3. high plasmid yield for large-scale production; and
4. improved purification efficiency of the screened subplasmids during the preparation process.

The technical solutions of the present invention are further illustrated in more detail by the examples and in conjunction with the accompanying drawings. Unless otherwise stated, the methods and materials in the examples described below are conventional products that can be purchased from the market. Those skilled in the art of the present invention would understand that the methods and materials described below are exemplary only and should not be considered as limiting the scope of the present invention.

### Example 1: Establishment of a mutant Π protein manipulation expression system in Escherichia coli

In the international application PCT/CN2021/133141 "PLASMID SYSTEM WITHOUT SELECTABLE MARKERS AND PRODUCTION METHOD THEREOF" filed by the inventor(s) on November 25, 2021, an engineered *Escherichia coli* strain JM108 containing a site-specific recombinase-inducible expression system (JM108-*araBAD*-*cre*engineered bacteria, see PCT/CN2021/133141, Example 1) was constructed. Brief preparation process of the JM108*-araBAD-cre* engineered bacteria:
1. the *cre* fragment (SEQ ID NO: 13) was synthesized by NANJING GENSCRIPT BIOTECH CO., LTD.;
2. the pSC101-araBAD linear fragment was obtained by treatment of the pKD46 plasmid with the restriction enzyme EcoR I. See NCBI Sequence ID: AY048746.1 for the pKD46 plasmid;
3. assembling was performed to obtain pSC101*-araBAD-cre*(*Amp^{R}*) plasmid containing a recombinase Cre inducible expression system; and
4. the recombinase system was integrated into the JM108 genome for expression by λRed recombination and CRISPR/Cas9 editing.

In this example, a mutant II protein manipulation expression system was constructed on the basis of this engineered strain. The system comprises: a regulatory region of a *Lac* operon, including a promoter P_{lacUV5-tac} and a LacOoperator sequence (with nucleotide sequence of the operator sequence as shown in SEQ ID NO: 1), a mutant II protein coding gene *pir116* (with sequence information as shown in SEQ ID NO: 2), and a transcription terminator. As a control group, the JM108*-araBAD-cre-pirWT* engineered strain (for *pirWT* sequence information, see SEQ ID NO:3) was also constructed through the same construction process as that for JM108*-araBAD-cre-pir116.*

The *pir-116* expression cassette was constructed onto the pKD46 vector plasmid. Using this plasmid as a template, the expression cassette was integrated into the JM108-*araBAD-cre* engineered bacteria genome by λRed recombination and CRISPR/Cas9 editing for expression. The specific steps were as follows:
1.1 Construction of *pKD46-Lac-pir116* plasmid
(1) Amplification of *pir116* gene (with pir1 competent strain provided by GenScript's reagent department as a template), *Lac* operon (including repressor protein coding genes, promoters and operator genes), and pKD46 vector (plasmid NCBI Sequence ID: AY048746.1). The amplification system and PCR system are shown in Table 1 and Table 2. The fragment sizes are 918 bp, 1512 bp and 3361 bp, respectively. The agarose gel results are shown in FIG. 2. The fragment size is correct and a gel recovery kit is used to recover the amplification products. Primer Star GXL DNA polymerase was purchased from TAKARA BIOTECHNOLOGY (BEIJING) CO., LTD. under Cat. No.: R050A.

**Table 1 Amplification system**

| System | Volume |
|---|---|
| Primer star GXL polymerase (2 U/µL) | 1 µL |
| Reaction buffer (5×) | 10 µL |
| dNTP (10 nM) | 5 µL |
| Primer-F | 1 µL |
| Primer-R | 1 µL |
| DNA template | 20 ng |
| H₂O | Adding to 50 µL |

**Table 2 Amplification process**

| No. | Procedures |
|---|---|
| 1 | 98°C for 2 min |
| 2 | 98°C for 30 s |
| 3 | 62°C/51°C/62°C for 30 s |
| 4 | 68°C for 3 min |
| 5 | 32 cycles from step 2 |
| 6 | 68°C for 5 min |

(2) Assembling of the *pKD46-Lac-pir116* plasmid
The assembling system is shown in Table 3 with reacting for 20 min at 50°C, the product was transformed into JM108 competent cells and then cultured on a transformation plate in a 37°C incubator. The Gene builder^{™} cloning Kit was purchased from NANJING GENSCRIPT BIOTECH CO., LTD. under Cat. No.: C20012009.

**Table 3 Assembling system**

| System | Volume |
|---|---|
| Gene builder 2×Master Mix recombinase | 10 µL |
| *pir-116* gene fragment | 1 µL |
| *Lac* operon fragment | 3.5 µL |
| pKD46 linear vector fragment | 5 µL |
| H₂O | 0.5 µL |

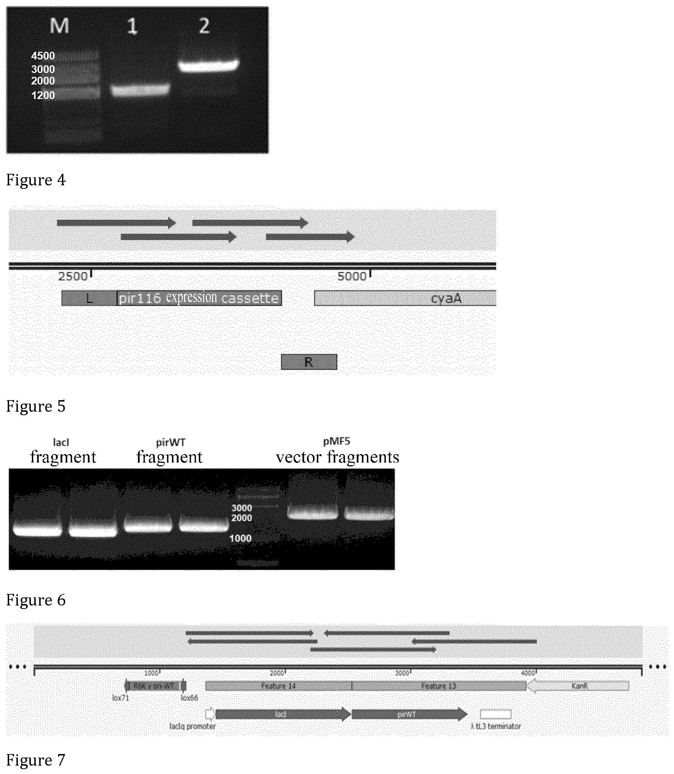

(3) Validation of the *pKD46-Lac-pir116* plasmid
10 single colonies were picked on the transformation plates in the above step, cultured in a 48-well plate medium at 37°C, and the bacteria solution was sent to the Nanjing GenScript's sequencing department for sanger sequencing, with sequencing results shown in FIG. 3. The *pKD46-Lac-pir116* plasmid was assembled successfully.
1.2 Integration of the mutant II protein manipulation expression system into the JM108-araBAD-cre engineered strain genome by λRed recombination and CRISPR/Cas9 editing. The specific steps were as follows:
   1) CRISPR/Cas9 specific targets between *hemC and cyaA* genes were designed: the gRNA was 20 bp in length and the specific gRNA sequence was as shown in SEQ ID NO: 9;
   2) homologous arms on both sides of the insertion site between *hemC* and *cyaA* genes were designed: see sequences SEQ ID NOs: 4 and 5 for the specific sequences of left and right homologous arms;
   3) traceless knocking in of the *pir-116* cassette was performed on the *E.coli* JM108-araBAD-Cre strain using λRed recombination technology and CRISPR/Cas9 technology;
   4) colony PCR screening was performed to verify edited strains. Bacterial detection primers were designed at both ends of the *pir-116* gene expression cassette (with primer sequences as shown in SEQ ID NOs: 6 and 7). The theoretical size of the bands obtained by colony PCR after a successful knocking in was 2798 bp, and the theoretical size of the bands obtained by colony PCR after a failed knocking in was 1365 bp. Results are shown in FIG. 4, with lane 1 being the PCR amplification bands without knocking in of a target fragment and lane 2 being the PCR amplification bands with knocking in of a target fragment. The band size was correct; and
   5) correct clones were verified by PCR for sanger sequencing, and as a result, the sequencing results were correct with no mutations, see FIG. 5 for the results.

### Example 2: Construction of a precursor plasmid containing the R6K replicon and the pirWT expression cassette

The *ori_{R6Kγ}* plasmid mainly exists in a haploid form in case of amplifying in low copy number. In this example, a wild-type II protein encoded by the *pirWT* gene was used to regulate the replication of *ori_{R6Kγ},* so that a precursor plasmid containing *ori_{R6Kγ}* was replicated in low copy number before induced recombination, and the haploid form of the plasmid was maintained. The precursor empty plasmid pMF5*-lacI-pirWT-loxp* comprises: the DNA replication element *ori_{R6Kγ,}* the resistance screening tag gene *Kan^{R},* the *Lac* operon repressor protein coding gene *lacI* and the wild-type II protein coding gene *pirWT* expressed in tandem (the tandem genes have their expression initiated by *P_{lacl}q*), terminator, a pair of specific direct recombination sites lox71/lox66, and multiple cloning sites, with the replication element and the multiple cloning sites located between lox71/lox66 (see FIG. 1 for a diagram of the structure).
2.1 The construction of the precursor empty plasmid pMF5*-lacI-pirWT-loxp* was based on the pMF5-loxp-RFP plasmid in the international application PCT/CN2021/133141 "PLASMID SYSTEM WITHOUT SELECTABLE MARKERS AND PRODUCTION METHOD THEREOF" (see international application PCT/CN2021/133141, Example 6), wherein *ori_{R6K γ}* had a size of 389 bp (with sequence as shown in SEQ ID NO:8); the plasmid was synthesized by NANJING GENSCRIPT BIOTECH CO., LTD., with sequence as shown in SEQ ID NO: 12, comprising the DNA replication element *ori_{R6Kγ,}* the resistance screening tag gene *Kan^{R},* a pair of specific direct recombination sites lox71/lox66, and the insertion sequence RFP target gene, with the replication element and the insertion gene located between lox71/lox66.

The *Lac* operon repressor protein coding gene *lacI* fragment (using primers *lacI-F*: AAGTATATATGAGTAAACTTGGTCTGACAGGACACCATCGAATGGTGCAA (SEQ ID NO: 14) and *lacI-R*: GTTCACGTCCATCATGACCTTGAGTCTCATCTCGAGTCACTGCCCGCTTTCCAGTC (SEQ ID NO: 15)), the *pirWT* gene fragment (using primers pir-F: ATGAGACTCAAGGTCATGAT (SEQ ID NO: 16) and pir-R: TTTCATTTGATGCTCGATGAGTTTTTCTAAGGTTCTT (SEQ ID NO: 17)) and the pMF5-loxp vector fragment (using primers R6K WT-loxp-F:
TTAGAAAAACTCATCGAGCA (SEQ ID NO: 18) and R6K WT-loxp-R:CTGTCAGACCAAGTTTACTC (SEQ ID NO: 19)) were amplified by PCR, respectively, with fragment sizes of 1227 bp, 1415 bp and 2289 bp, respectively. The amplification system is shown in Table 4, the amplification procedure is shown in Table 5, and the results are shown in FIG. 6, with lanes 1 and 2 representing *lacI* amplification fragments, lanes 3 and 4 representing *pirWT* amplification fragments and lanes 6 and 7 representing pMF5 vector amplification fragments, and all of these fragments have the correct sizes and are recovered using a gel recovery kit. Assembling was performed with the kit Gene builder^{™} cloning Kit (with the assembling system as shown in Table 6) for 20 min at 50°C, and the resultant was transformed into JM108, then the transformed products were spread at 50 µL/100 µL and cultured at 37°C overnight.

**Table 4 Amplification system**

| System | Volume |
|---|---|
| | |
| Primer Star GXL polymerase (2 U/µL) | 1 µL |
| Reaction buffer (5×) | 10 µL |
| dNTP (10 mM) | 5 µL |
| DMSO | 2.5 µL |
| Primer-F (50 pmol) | 1 µL |
| Primer-R (50 pmol) | 1 µL |
| DNA template | 20 ng |
| H₂O | Adding to 50 µL |

**Table 5 Amplification process**

| No. | Procedures |
|---|---|
| 1 | 98°C for 2 min |
| 2 | 98°C for 30 s |
| 3 | 62°C/55°C/51°C for 30s |
| 4 | 68°C for 1 min 18 s/1 min 30 s/2 min 18 s |
| 5 | 32 cycles from step 2 |
| 6 | 68°C for 5 min |

**Table 6 Assembling system**

| System | Volume |
|---|---|
| Gene builder 2×Master Mix recombinase | 10 µL |
| *lacI* gene fragment (118 ng/µL) | 3 µL |
| *pirWT* gene fragment (108 ng/µL) | 4 µL |
| pMF5 vector fragment (119 ng/µL) | 3 µL |
| H₂O | 4.1 µL |

Single colonies were grown in the assembled plasmid pMF5-*lacI-pirWT-loxp,* and 8 single clones were picked for sanger sequencing. The results of the sanger sequencing are shown in FIG. 7 with a successful assembling.

### 2.2 Construction of precursor plasmid pMF5-lacI-pirWT-loxp

Based on the constructed precursor empty plasmid pMF5-*lacI-pirWT-loxp,* fragments of different sequence lengths were inserted at the multiple cloning site, with the gene fragments of 1.0 kb, 1.5 kb and 2.3 kb in length, respectively. Control plasmids were constructed simultaneously in this example: pMF5-loxp- fragments of different sequence lengths.

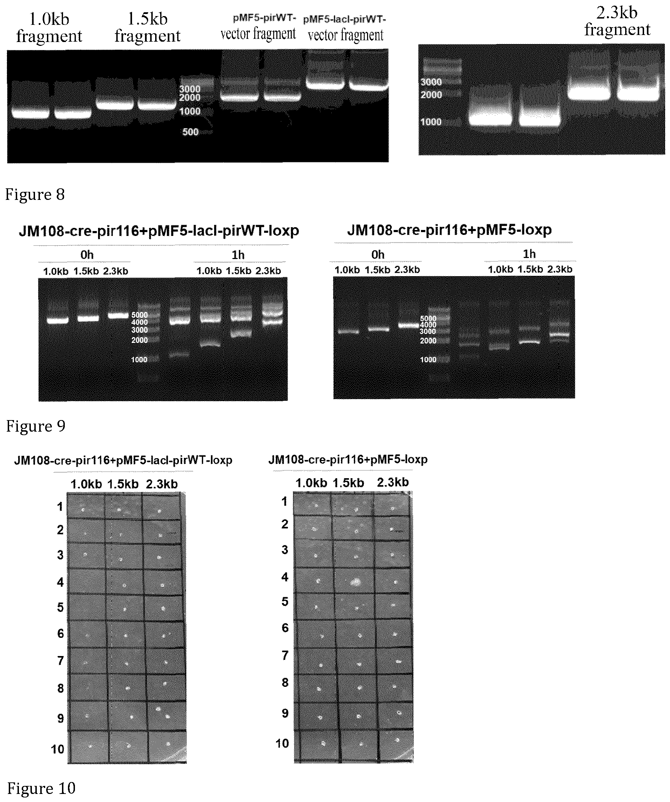

The above three gene fragments (gene fragment 1.0 kb, gene fragment 1.5 kb and gene fragment 2.3 kb), pMF5*-lacI-pirWT-loxp* vector fragment and pMF5-loxp vector fragment were amplified through PCR, with amplification system as shown in Table 7 and amplification procedures as shown in Table 8. The results are shown in FIG. 8 with correct sizes, and the fragments are recovered using a gel recovery kit. Assembling was performed with the kit Gene builder^{™} cloning Kit (with the assembling system as shown in Table 9) for 20 min at 50°C, and the resultant was transformed into JM108, then the transformed products were spread at 50 µL/100 µL and cultured at 37°C overnight.

**Table 7 Amplification system**

| System | Volume |
|---|---|
| Primer Star GXL polymerase (2 U/µL) | 1 µL |
| Reaction buffer (5×) | 10 µL |
| dNTP (10 mM) | 5 µL |
| DMSO | 2.5 µL |
| Primer-F (50 pmol) | 1 µL |
| Primer-R (50 pmol) | 1 µL |
| Plasmid template/gene fragment (20 ng/µL) | 0.3 µL |
| H₂O | 29.2 µL |

**Table 8 Amplification process**

| No. | Procedures |
|---|---|
| 1 | 98°C for 2 min |
| 2 | 98°C for 30 s |
| 3 | 62°C/55°C for 30s |
| 4 | 68°C for 2 min 18 s/4 min 54s |
| 5 | 32 cycles from step 2 |
| 6 | 68°C for 5 min |

**Table 9 Assembling system**

| System | Volume |
|---|---|
| Gene builder 2×Master Mix recombinase | 10 µL |
| Fragments of different sequence lengths pMF5*-lacI-pirWT-loxp* vector fragment / *pMF5-loxp* vector fragment | n_{fragment} : n_{vector} = 2 : 1 |
| H₂O | Adding to 20 µL |

Single colonies were grown in the assembled pMF5*-lacI-pirWT-loxp-* fragments of different sequence lengths and pMF5-loxp- fragments of different sequence lengths, and 8 single clones were picked each for sanger sequencing. The results of the sanger sequencing show a successful assembling.

### Example 3: Preparation of a resistance-screening-tag-free plasmid based on the wild-type-mutant Π protein switching expression system

During the preparation and purification processes of a resistance-screening-tag-free plasmid, a high copy precursor plasmid was selected, resulting in heavy contamination of multimers and low efficiency in screening for resistance-free strains; and when a low copy precursor plasmid was selected, the yield for a large-scale preparation was insufficient and genome contamination was severe. This example expounds a method of successfully improving purification efficiency and plasmid yield in the preparation of a resistance-screening-tag-free plasmid using a wild-type-mutant II protein switching expression system by means of multiple controls. The specific operations were as follows:
3.1 Genetically modified strain *JM108-cre-pir116* was taken for preparing competent cells, and the pMF5*-lacI-pirWT-loxp* group precursor plasmid was taken for transformation. The insertion fragment sequences were fragments of 1.0 kb, 1.5 kb and 2.3 kb, respectively, and coating on a Kan resistance plate was performed. Control group: JM108*-cre-pirl16+*pMF5-loxp group precursor plasmid. The specific transformation steps were as follows:
   1) taking the competent cells and placing same on ice to melt naturally;
   2) adding 1 µL of plasmid to the competent cells on ice and mixing well the competent cells with the plasmid;
   3) incubating same in an ice bath for 30 min;
   4) performing heat shock at 42°C for 90 s and incubating same in an ice bath for 3 min;
   5) adding 800 µL of fresh LB liquid medium for incubating for 45 min in a full-temperature shaking incubator at 37°C and 220 rpm;
   6) applying an appropriate amount of bacteria solution to a solid plate of LB+Kan+1% glucose and culturing the plate overnight at 37°C in an incubator; and
   7) picking single colonies of normal size for subsequent experiments.
3.2 Addition of arabinose to induce Cre-loxp recombination:

An amount of arabinose was added to the culture system to induce the expression of a Cre recombinase to effect Cre-loxp recombination. The specific operation was as follows:
1) picking 3-4 transformed clones and placing them in a 4 mL LB+ Kan+1% glucose liquid medium for culturing overnight at 37°C and 220 rpm to enrich for bacteria;
2) sub-packaging 4 mL of bacteria solution into 2 EP tubes of 2 mL equally, resining 1 tube twice with resistance-free LB medium, and resuspending the bacteria in an inducer solution (the inducer solution is LB+2% arabinose);
3) inducing recombination at 37°C and 220 rpm for 1 h; and
4) taking 5 µL of the recombined bacteria solution for streaking four-way on a resistance-free LB solid plate and culturing the plate overnight at 37°C in an incubator. The remaining bacteria solution and the another 1 tube of bacteria solution without induced recombination were subjected to plasmid extraction and verification. Agarose gel electrophoresis was carried out, and the electrophoresis results of plasmid verification after induced recombination were shown in FIG. 9, with plasmids without induced recombination as a control. On the left side of FIG. 9 is the group *JM108-cre-pir116+* pMF5*-lacI-pirWT-loxp,* and the 1.0 kb, 1.5 kb and 2.3 kb on the left side of Marker are pMF5-*lacI-pirWT-loxp*-1.0 kb/1.5 kb/2.3 kb precursor plasmids without induced recombination, respectively, having a size of 5817 bp, 6317 bp and 7078 bp, respectively; the 1.0kb, 1.5kb and 2.3kb on the right side of Marker are pMF5*-lacI-pirWT-loxp*-1.0 kb/1.5 kb/2.3 kb resistance-screening-tag-free plasmids obtained upon 1 h of induced recombination, respectively, having a size of 1429 bp, 1929 bp and 2684 bp, respectively, wherein the induced recombination product on the right side should be 4422 bp in size, having an additional replication-incompetent circular double-stranded DNA in addition to the precursor plasmid and the resistance-screening-tag-free plasmid, compared to the products obtained without induced recombination on the left side. All bands have correct size.

On the right side of FIG. 9 is the group *JM108-cre-pir116+ pMF5-loxp,* and the 1.0 kb, 1.5 kb and 2.3 kb on the left side of Marker are pMF5-loxp-1.0 kb/1.5 kb/2.3 kb precursor plasmids without induced recombination, respectively, having a size of 3265 bp, 3765 bp and 4526 bp, respectively; the 1.0kb, 1.5kb and 2.3kb on the right side of Marker are pMF5-*loxp-1.0* kb/1.5 kb/2.3 kb resistance-screening-tag-free plasmids obtained upon 1 h of induced recombination, respectively, having a size of 1429 bp, 1929 bp and 2690 bp, respectively, wherein the induced recombination product on the right side should be 1836 bp in size, having an additional replication-incompetent circular double-stranded DNA in addition to the precursor plasmid and the resistance-screening-tag-free plasmid, compared to the products obtained without induced recombination on the left side. All bands have correct size.
3.3 Kan resistance screening, and purification to obtain a resistance-screening-tag-free plasmid with the following steps:
1) picking 10 single colonies each from the resistance-free plates coated with the above recombined bacteria solution, and spotting each colony on an LB+Kan (1‰) resistance plate and incubating the plate overnight at 37°C in an incubator (resistance screening plates are shown in FIG. 10); meanwhile, inoculating an LB resistance-free liquid medium with the colony for culturing at 37°C and 220 rpm for resistance screening;
2) picking out the clones which had no colony growth on the LB+Kan resistance plate, but had normal growth in the resistance-free LB liquid medium, extracting the plasmid and verifying the plasmid; and
3) carrying out agarose gel electrophoresis on the extracted plasmid, and taking the plasmid with the correct plasmid size as the resistance-screening-tag-free plasmid product.

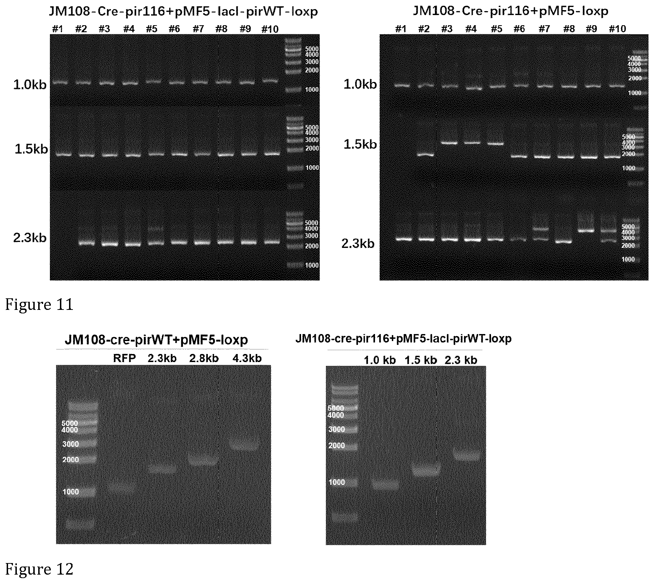

As shown in FIG. 10, the resistance of #4 clone in *JM108-cre-pir116+ pMF5-loxp-*1.5kb group is not lost, and none of the remaining clones grows on the resistance plate, so the resistance gene is lost; and none of the clones in JM108*-cre-pir116+* pMF5*-lacI-pirWT-loxp* group grows on the resistance plate. In FIG. 11, the plasmid of *JM108-cre-pir116+* pMF5*-lacI-pirWT-loxp* group on the left side corresponds to the clone with lost resistance on the left side in FIG. 10. All the main bands of the plasmids for the clones in this group are haploid bands with high purity and correct size. In *JM108-cre-pir116+* pMF5 *-loxp* group on the right side, all the main bands in the 1.0 kb group are all of haploid size, with contamination due to a small amount of impure bands; one clone in the 1.5 kb group does not lose its resistance, and the plasmid band is not verified; the haploid clones account for 60%, and the haploid clones in the 2.3 kb group account for 60%. In comparison, the JM108-*cre-pir116+* pMF5*-lacI-pirWT-loxp* group and *JM108-cre-pir116+* pMF5 *-loxp* group have the same insertion fragments of 1.0 kb, 1.5 kb and 2.3 kb, but the results of the above examples prove that the *JM108-cre-pir116+* pMF5-*lacI-pirWT-loxp* group, i.e. the wild-type-mutant II protein switching expression system has effectively improved preparation and purification efficiency of an resistance-screening-tag-free plasmid.

### Example 4: Large-scale extraction of resistance-screening-tag-free plasmids

In this example, cell culture, large-scale plasmid extraction and QC analysis were carried out on the basis of the resistance-screening-tag-free plasmid obtained in Example 3, with JM108-*cre-pirWT+*PMF5*-loxp* group as the control, but not limited to this examplification. The cell culture and large-scale plasmid extraction were completed by

### NANJING GENSCRIPT BIOTECH CO., LTD.

An 100 mL rich medium was inoculated with strains containing only the pure resistance-screening-tag-free plasmid obtained by the above steps of recombination, verification, etc. for culture overnight. Bacteria were collected for lysis, neutralizing and purifying, and finally the extracted plasmids were obtained.

### QC detection results:

sanger sequencing was performed by NANJING GENSCRIPT BIOTECH CO., LTD. on the products obtained from the large-scale plasmid extraction, and the sequencing result of a resistance-gene-free sequence was correct. The plasmid validation results are shown in FIG. 12 and the plasmid yields are shown in Table 10. The JM108*-cre-pirWT+*pMF5*-loxp* group (the inserted gene fragments are 2.3 kb, 2.8 kb and 4.3 kb, respectively) has unique plasmid bands and correct sizes, but the low copy number of plasmids leads to low yield and genome contamination. All plasmid bands in JM108*-cre-pir116+*pMF5-*lacI-pirWT-loxp* group (the inserted gene fragments are 1.0 kb, 1.5 kb and 2.3 kb, respectively) are unique and correct in size, and the plasmid yield is one order of magnitude higher than that in JM108*-cre-pirWT+*pMF5*-loxp* group.

The results of screening and purification efficiency and large-scale plasmid extraction yield in the process of recombination show that the wild-type-mutant II protein switching expression system has the advantages of high screening and purification efficiency and high large-scale plasmid extraction yield, and has successfully achieved the goals of reducing the proportion of sequences of bacterial origin in the product plasmid, reducing potential safety risks and improving plasmid yield.

**Table 10 Plasmid yield**

| **JM108*-cre-pirWT+*pMF5*-loxp* (mg/L)** | | **JM108-*cre-pir116+*pMF5-*lacI-pirWT-loxp* (mg/L)** | |
|---|---|---|---|
| **RFP** | **0.4** | **1.0kb** | **1.6** |
| **2.3kb** | **0.1** | **1.5kb** | **2.2** |
| **2.8kb** | **0.2** | **2.3kb** | **3.3** |
| **4.3kb** | **0.3** | | |

The partial sequence information referred to herein is as follows:
*lacO* operator sequence (SEQ ID NO: 1): 
TTTACACTTTATGCTTCCGGCTCGTATAATGTGTGGAATTGTGAGCGGATAACAATTTCACACAG
GAAACAGGATCGATCCATCGATGAGCTTACTCCCCATCCCCCTGTTGACAATTAATCATCGGCTCG
TATAATGTGTGGAATTGTGAGCGGATAACAATTTCACACAGGAAACAGGATCAGCTTACTCCCCA
TCCCCCTG
Mutant II protein coding gene *pir-116* (SEQ ID NO: 2): 
ATGAGACTCAAGGTCATGATGGACGTGAACAAAAAAACGAAAATTCGCCACCGAAACGAGCTAAA
TCACACCCTGGCTCAACTTCCTTTGCCCGCAAAGCGAGTGATGTATATGGCGCTTGCTCCCATTGA
TAGCAAAGAACCTCTTGAACGAGGGCGAGTTTTCAAAATTAGGGCTGAAGACCTTGCAGCGCTCG
CCAAAATCACCCCATCGCTTGCTTATCGACAATTAAAAGAGGGTGGTAGATTACTTGGTGCCAGC
AAAATTTCGCTAAGAGGGGATGATATCATTGCTTTAGCTAAAGAGCTTAACCTGCTCTTTACTGC
TAAAAACTCCCCTGAAGAGTTAGATCTTAACATTATTGAGTGGATAGCTTATTCAAATGATGAAG
GATACTTGTCTTTAAAATTCACCAGAACCATAGAACCATATATCTCTAGCCTTATTGGGAAAAAA
AATAAATTCACAACGCAATTGTTAACGGCAAGCTTACGCTTAAGTAGCCAGTATTCATCTTCTCT
TTATCAACTTATCAGGAAGCATTACTCTAATTTTAAGAAGAAAAATTATTTTATTATTTCCGTTG
 
ATGAGTTAAAGGAAGAGTTAATAGCTTATACTTTTGATAAAGATGGAAATATTGAGTACAAATA
CCCTGACTTTCCTATTTTTAAAAGGGATGTGTTAAATAAAGCCATTGCTGAAATTAAAAAGAAAA
CAGAAATATCGTTTGTTGGCTTCACTGTTCATGAAAAAGAAGGAAGAAAAATTAGTAAGCTGAA
GTTCGAATTTGTCGTCGATGAAGATGAATTTTCTGGCGATAAAGATGATGAAGCTTTTTTTATGA
ATTTATCTGAAGCTGATGCAGCTTTTCTCAAGGTATTTGATGAAACCGTACCTCCCAAAAAAGCT
AAGGGGTAA
II protein coding gene *pirWT* (SEQ ID NO: 3): 
ATGAGACTCAAGGTCATGATGGACGTGAACAAAAAAACGAAAATTCGCCACCGAAACGAGCTAAA
TCACACCCTGGCTCAACTTCCTTTGCCCGCAAAGCGAGTGATGTATATGGCGCTTGCTCCCATTGA
TAGCAAAGAACCTCTTGAACGAGGGCGAGTTTTCAAAATTAGGGCTGAAGACCTTGCAGCGCTCG
CCAAAATCACCCCATCGCTTGCTTATCGACAATTAAAAGAGGGTGGTAAATTACTTGGTGCCAGC
AAAATTTCGCTAAGAGGGGATGATATCATTGCTTTAGCTAAAGAGCTTAACCTGCCCTTTACTGC
TAAAAACTCCCCTGAAGAGTTAGATCTTAACATTATTGAGTGGATAGCTTATTCAAATGATGAAG
GATACTTGTCTTTAAAATTCACCAGAACCATAGAACCATATATCTCTAGCCTTATTGGGAAAAAA
AATAAATTCACAACGCAATTGTTAACGGCAAGCTTACGCTTAAGTAGCCAGTATTCATCTTCTCT
TTATCAACTTATCAGGAAGCATTACTCTAATTTTAAGAAGAAAAATTATTTTATTATTTCCGTTG
ATGAGTTAAAGGAAGAGTTAATAGCTTATACTTTTGATAAAGATGGAAATATTGAGTACAAATA
CCCTGACTTTCCTATTTTTAAAAGGGATGTGTTAAATAAAGCCATTGCTGAAATTAAAAAGAAAA
CAGAAATATCGTTTGTTGGCTTCACTGTTCATGAAAAAGAAGGAAGAAAAATTAGTAAGCTGAA
GTTCGAATTTGTCGTTGATGAAGATGAATTTTCTGGCGATAAAGATGATGAAGCTTTTTTTATGA
ATTTATCTGAAGCTGATGCAGCTTTTCTCAAGGTATTTGATGAAACCGTACCTCCCAAAAAAGCT
AAGGGGTAA
Left homologous arm (SEQ ID NO: 4): 
TGCCGGTAACGCATCCAGACTGTCATAGTTATTGGACACAAAGGCATCGCGAGGATCTTCACGCT
CACAAATAGTGACCAGTCCCAGACCTTGCGGGAATTCAACCGGCACATCTTTCATTGAGTGTACG
GCGATATCGGCGCGATTTTCGAGGAGCGCGACTTCCAGCTCTTTTACAAATAAGCCTTTTCCGCCT
ACTTTCGCCAGCGGCGTATCAAGAATCACATCGCCGCGCGTCACCATCGGTACCAGTTCAACGACC
AGGCCCGGATGGCTCGCCATCAACTTGTCTTTGACATAGTGTGCCTGCCAGAGTGCAAGTGGGCT
TTGGCGTGTGGCAATTCTTAAAACATTGTCTAACATGCTTGTTACCGTCATTATCATCCGTGGTC
CATCCTAACATCCTTGCCAGAGTGATGTCAGTGTTGTGGTGAAACGTAGACGCCTGCGCAAACCG
TAAAATGAGGTCTGGCAGTGGATCCTGACAGGCGTTTCACG
Right homologous arm (SEQ ID NO: 5): 
TGCTACACTTGTATGTAGCGCATCTTTCTTTACGGTCAATCAGCAAGGTGTTAAATTGATCACGT
TTTAGACCATTTTTTCGTCGTGAAACTAAAAAAACCAGGCGCGAAAAGTGGTAACGGTTACCTTT
GACATACGAAATATCCCGAATGCCGCGTGTTACCGTTGATGTTGGCGGAATCACAGTCATGACGG
GTAGCAAATCAGGCGATACGTCTTGTACCTCTATATTGAGACTCTGAAACAGAGACTGGATGCCA
TAAATCAATTGCGTGTGGATCGCGCGCTTGCTGCTATGGGGCCTGCATTCCAACAGGTCTACAGT
CTACTGCCGACATTGTTGCACTATCACCATCCGCTAATGCCGGGTTACCTTGATGGTAACGTTCCC
AAAGGCATTTGCCTTTACACGCCTGATGAAACTCAACGCCACTACCTGAACGAGCTTGAACTGTA
TCGTGGAATGTCAGTACAGGATCCGCCGAAAGGTG
C4241GG050-1JJFH (SEQ ID NO: 6): TTAGTCCGGCTACGGCAAGAATGAT
C4241GG050-1JJRH (SEQ ID NO: 7): GCTGACTTCCACACCCAGCGAGGCG
*ori_{R6Kγ}* (SEQ ID NO: 8): 
TGTCAGCCGTTAAGTGTTCCTGTGTCACTCAAAATTGCTTTGAGAGGCTCTAAGGGCTTCTCAGT
GCGTTACATCCCTGGCTTGTTGTCCACAACCGTTAAACCTTAAAAGCTTTAAAAGCCTTATATAT
TCTTTTTTTTCTTATAAAACTTAAAACCTTAGAGGCTATTTAAGTTGCTGATTTATATTAATTTT
ATTGTTCAAACATGAGAGCTTAGTACGTGAAACATGAGAGCTTAGTACGTTAGCCATGAGAGCTT
AGTACGTTAGCCATGAGGGTTTAGTTCGTTAAACATGAGAGCTTAGTACGTTAAACATGAGAGCT
TAGTACGTGAAACATGAGAGCTTAGTACGTACTATCAACAGGTTGAACTGCTGATCTTCAGATC
gRNA sequence (SEQ ID NO: 9): TTGTAATAAGGAATTTACAG
Lox71 (SEQ ID NO: 10): TACCGTTCGTATAATGTATGCTATACGAAGTTAT
Lox66 (SEQ ID NO :11): ATAACTTCGTATAATGTATGCTATACGAACGGTA
*pMF5-loxp-RFP* plasmid (SEQ ID NO:12): 
TTCTCATGTTTGACAGCTTATCATCGATAAGCTTTAATGCGGTAGTTTATCACAGTTAAATTGCT
AACGCAGTCAGGCACCGTGTATGGAAGCCGGCGGCACCTCGCTAACGGATTCACCACTCCAAGAA
TTGGAGCCAATCAATTCTTGCGGAGAACTGTGAATGCGCAAACCAACCCTTGGCAGAACATATCC
ATCGCGTCCGCCATCTCCAGCAGCCGCACGCGGCGCATCTCGGGCAGCGTTGGGTCCTGGCCACGG
GTGCGCATGATCGTGCTCCTGTCGTTGAGGACCCGGCTAGGCTGGCGGGGTTGCCTTACTGGTTA
GCAGAATGAATCACCGATACGCGAGCGAACGTGAAGCGACTGCTGCTGCAAAACGTCTGCGACCT
GAGCAACAACATGAATGGTCTTCGGTTTCCGTGTTTCGTAAAGTCTGGAAACGCGGAAGTCAGCG
CCCTGCACCATTATGTTCCGGATCTGCATCGCAGGATGCTGCTGGCTACCCTGTGGAACACCTACA
TCTGTATTAACGAAGCGCTGGCATTGACCCTGAGTGATTTTTCTCTGGTCCCGCCGCATCCATACC
GCCAGTTGTTTACCCTCACAACGTTCCAGTAACCGGGCATGTTCATCATCAGTAACCCGTATCGTG
AGCATCCTCTCTCGTTTCATCGGTATCATTACCCCCATGAACAGAAATCCCCCTTACACGGAGGCA
TCAATAACTTCGTATAATGTATGCTATACGAACGGTACTGCAGTGTCAGCCGTTAAGTGTTCCTG
 
TGTCACTCAAAATTGCTTTGAGAGGCTCTAAGGGCTTCTCAGTGCGTTACATCCCTGGCTTGTTG
TCCACAACCGTTAAACCTTAAAAGCTTTAAAAGCCTTATATATTCTTTTTTTTCTTATAAAACTT
AAAACCTTAGAGGCTATTTAAGTTGCTGATTTATATTAATTTTATTGTTCAAACATGAGAGCTTA
GTACGTGAAACATGAGAGCTTAGTACGTTAGCCATGAGAGCTTAGTACGTTAGCCATGAGGGTTT
AGTTCGTTAAACATGAGAGCTTAGTACGTTAAACATGAGAGCTTAGTACGTGAAACATGAGAGCT
TAGTACGTACTATCAACAGGTTGAACTGCTGATCTTCAGATCGGTACCCAATACGCAAACCGCCT
CTCCCCGCGCGTTGGCCGATTCATTAATGCAGCTGGCACGACAGGTTTCCCGACTGGAAAGCGGGC
AGTGAGCGCAACGCAATTAATGTGAGTTAGCTCACTCATTAGGCACCCCAGGCTTTACACTTTAT
GCTTCCGGCTCGTATGTTGTGTGGAATTGTGAGCGGATAACAATTTCACACATACTAGAGAAAGA
GGAGAAATACTAGATGGCTTCCTCCGAAGACGTTATCAAAGAGTTCATGCGTTTCAAAGTTCGTA
TGGAAGGTTCCGTTAACGGTCACGAGTTCGAAATCGAAGGTGAAGGTGAAGGTCGTCCGTACGAA
GGTACCCAGACCGCTAAACTGAAAGTTACCAAAGGTGGTCCGCTGCCGTTCGCTTGGGACATCCT
GTCCCCGCAGTTCCAGTACGGTTCCAAAGCTTACGTTAAACACCCGGCTGACATCCCGGACTACCT
GAAACTGTCCTTCCCGGAAGGTTTCAAATGGGAACGTGTTATGAACTTCGAAGACGGTGGTGTTG
TTACCGTTACCCAGGACTCCTCCCTGCAAGACGGTGAGTTCATCTACAAAGTTAAACTGCGTGGT
ACCAACTTCCCGTCCGACGGTCCGGTTATGCAGAAAAAAACCATGGGTTGGGAAGCTTCCACCGA
ACGTATGTACCCGGAAGACGGTGCTCTGAAAGGTGAAATCAAAATGCGTCTGAAACTGAAAGACG
GTGGTCACTACGACGCTGAAGTTAAAACCACCTACATGGCTAAAAAACCGGTTCAGCTGCCGGGT
GCTTACAAAACCGACATCAAACTGGACATCACCTCCCACAACGAAGACTACACCATCGTTGAACA
GTACGAACGTGCTGAAGGTCGTCACTCCACCGGTGCTTAATAACGCTGATAGTGCTAGTGTAGAT
CGCTACTAGAGCCAGGCATCAAATAAAACGAAAGGCTCAGTCGAAAGACTGGGCCTTTCGTTTTA
TCTGTTGTTTGTCGGTGAACGCTCTCTACTAGAGTCACACTGGCTCACCTTCGGGTGGGCCTTTCT
GCGTTTATAGTCGACTCGAGCGGCCAGTCGACGCATGCTACCGTTCGTATAATGTATGCTATACG
AAGTTATTGATCTTTTCTACGGGGTCTGACGCTCAGTGGAACGAAAACTCACGTTAAGGGATTTT
GGTCATGAGATTATCAAAAAGGATCTTCACCTAGATCCTTTTAAATTAAAAATGAAGTTTTAAAT
CAATCTAAAGTATATATGAGTAAACTTGGTCTGACAGTTAGAAAAACTCATCGAGCATCAAATGA
AACTGCAATTTATTCATATCAGGATTATCAATACCATATTTTTGAAAAAGCCGTTTCTGTAATGA
AGGAGAAAACTCACCGAGGCAGTTCCATAGGATGGCAAGATCCTGGTATCGGTCTGCGATTCCGA
CTCGTCCAACATCAATACAACCTATTAATTTCCCCTCGTCAAAAATAAGGTTATCAAGTGAGAAA
TCACCATGAGTGACGACTGAATCCGGTGAGAATGGCAAAAGTTTATGCATTTCTTTCCAGACTTG
TTCAACAGGCCAGCCATTACGCTCGTCATCAAAATCACTCGCATCAACCAAACCGTTATTCATTCG
TGATTGCGCCTGAGCGAGACGAAATACGCGATCGCTGTTAAAAGGACAATTACAAACAGGAATCG
AATGCAACCGGCGCAGGAACACTGCCAGCGCATCAACAATATTTTCACCTGAATCAGGATATTCT
 
TCTAATACCTGGAATGCTGTTTTCCCGGGGATCGCAGTGGTGAGTAACCATGCATCATCAGGAGT
ACGGATAAAATGCTTGATGGTCGGAAGAGGCATAAATTCCGTCAGCCAGTTTAGTCTGACCATCT
CATCTGTAACATCATTGGCAACGCTACCTTTGCCATGTTTCAGAAACAACTCTGGCGCATCGGGCT
TCCCATACAATCGATAGATTGTCGCACCTGATTGCCCGACATTATCGCGAGCCCATTTATACCCAT
ATAAATCAGCATCCATGTTGGAATTTAATCGCGGCCTAGAGCAAGACGTTTCCCGTTGAATATGG
CTCATCACATTTCCCCGAAAAGTGCCACCTGACGTCTAAGAAACCATTATTATCATGACATTAAC
CTATAAAAATAGGCGTATCACGAGGCCCTTTCGTCTTCAAGAA
Cre recombinase coding sequence (SEQ ID NO: 13): 
CACCATCACCATCACCATATGTCCAATTTACTGACCGTACACCAAAATTTGCCTGCATTACCGGTC
GATGCAACGAGTGATGAGGTTCGCAAGAACCTGATGGACATGTTCAGGGATCGCCAGGCGTTTTC
TGAGCATACCTGGAAAATGCTTCTGTCCGTTTGCCGGTCGTGGGCGGCATGGTGCAAGTTGAATA
ACCGGAAATGGTTTCCCGCAGAACCTGAAGATGTTCGCGATTATCTTCTATATCTTCAGGCGCGC
GGTCTGGCAGTAAAAACTATCCAGCAACATTTGGGCCAGCTAAACATGCTTCATCGTCGGTCCGG
GCTGCCACGACCAAGTGACAGCAATGCTGTTTCACTGGTTATGCGGCGGATCCGAAAAGAAAACG
TTGATGCCGGTGAACGTGCAAAACAGGCTCTAGCGTTCGAACGCACTGATTTCGACCAGGTTCGT
TCACTCATGGAAAATAGCGATCGCTGCCAGGATATACGTAATCTGGCATTTCTGGGGATTGCTTA
TAACACCCTGTTACGTATAGCCGAAATTGCCAGGATCAGGGTTAAAGATATCTCACGTACTGACG
GTGGGAGAATGTTAATCCATATTGGCAGAACGAAAACGCTGGTTAGCACCGCAGGTGTAGAGAAG
GCACTTAGCCTGGGGGTAACTAAACTGGTCGAGCGATGGATTTCCGTCTCTGGTGTAGCTGATGA
TCCGAATAACTACCTGTTTTGCCGGGTCAGAAAAAATGGTGTTGCCGCGCCATCTGCCACCAGCC
AGCTATCAACTCGCGCCCTGGAAGGGATTTTTGAAGCAACTCATCGATTGATTTACGGCGCTAAG
GATGACTCTGGTCAGAGATACCTGGCCTGGTCTGGACACAGTGCCCGTGTCGGAGCCGCGCGAGA
TATGGCCCGCGCTGGAGTTTCAATACCGGAGATCATGCAAGCTGGTGGCTGGACCAATGTAAATA
TTGTCATGAACTATATCCGTAACCTGGATAGTGAAACAGGGGCAATGGTGCGCCTGCTGGAAGAT
GGCGATTAA
*P_{lacl}q* promoter sequence (SEQ ID NO: 20)

GACACCATCGAATGGTGCAAAACCTTTCGCGGTATGGCATGATAGCGCCCGGAAGAGAGTCAATT
CAGGGTGGTGAAT

The embodiments of the present invention are not limited to the above-mentioned examples, and various modifications and improvements in forms and details may be made to the present invention by those of ordinary skill in the art without departing from the spirit and scope of the present invention, and the modifications and improvements are considered to fall within the scope of protection of the present invention.

## Claims

1. A precursor plasmid comprising:
1) a conditioned replication initiation site having a plasmid replication initiation capacity that is dependent on regulatory proteins, wherein the conditioned replication initiation site has a first replication initiation state in the presence of a first regulatory protein and a second replication initiation state in the presence of a second regulatory protein, and has a stronger ability to initiate plasmid replication in the second replication initiation state than in the first replication initiation state;
2) a first regulatory protein expression cassette expressing the first regulatory protein;
3) a sequence encoding a repressor protein;
4) a screened tag gene;
5) a target gene, or a cloning site for inserting the target gene; and
6) paired recombination sites,
wherein the paired recombination sites enable the self-recombination of the precursor plasmid in the presence of a recombinase to form a subplasmid and a circular double-stranded DNA,
wherein the subplasmid comprises the conditioned replication initiation site and the cloning site, or comprises the conditioned replication initiation site and the target gene, and the circular double-stranded DNA comprises the screened tag gene, the first regulatory protein expression cassette, and the sequence encoding a repressor protein.

2. The precursor plasmid of claim 1, wherein the presence of the repressor protein enables the inhibition of expression of the second regulatory protein in a host cell expressing the second regulatory protein upon introduction of the precursor plasmid into the host cell.

3. The precursor plasmid of claim 1 or 2, wherein the host cell comprises a second regulatory protein expression cassette expressing a second regulatory protein, wherein the second regulatory protein expression cassette comprises an expression regulatory sequence, and when bound to the expression regulatory sequence, the repressor protein inhibits the expression of the second regulatory protein.

4. The precursor plasmid of any one of claims 1-3, wherein the sequence encoding a repressor protein is located in the first regulatory protein expression cassette such that the repressor protein is expressed in tandem with the first regulatory protein.

5. The precursor plasmid of any one of claims 1-4, wherein the conditioned replication initiation site is the *ori_{R6Kγ}* replication initiation site.

6. The precursor plasmid of any one of claims 1-5, wherein the first regulatory protein is a wild-type II protein and the second regulatory protein is a mutant of the wild-type II protein.

7. The precursor plasmid of any one of claims 1-6, wherein the second regulatory protein is a product of *pir-116* coding gene.

8. The precursor plasmid of any one of claims 1-7, wherein the *pir-116* coding gene comprises the sequence as shown in SEQ ID NO: 2.

9. The precursor plasmid of any one of claims 1-8, wherein the conditioned replication initiation site comprises the sequence as shown in SEQ ID NO: 8, or a nucleotide sequence having at least 80% identity to the sequence as shown in SEQ ID NO: 8 and capable of exerting the replication initiation function.

10. The precursor plasmid of any one of claims 1-9, wherein the sequences of the paired recombination sites are direct sequences.

11. The precursor plasmid of any one of claims 1-10, wherein the paired recombination sites are direct loxP sequences, and the recombinase is a Cre recombinase; the paired recombination sites are direct FRT sequences, and the recombinase is a Flp recombinase; or the paired recombination sites are direct attB/attP sequences, and the recombinase is a PhiC31 recombinase.

12. The precursor plasmid of any one of claims 1-11, wherein the paired recombination sites are direct lox71 and lox66 sequences.

13. The precursor plasmid of any one of claims 1-12, wherein the repressor protein is LacI protein.

14. The precursor plasmid of any one of claims 3-13, wherein the expression regulatory sequence comprises a *lacO* operator sequence.

15. The precursor plasmid of claim 14, wherein the *lacO* operator sequence comprises the sequence as shown in SEQ ID NO: 1.

16. The precursor plasmid of any one of claims 1-15, wherein the promoter of the first regulatory protein expression cassette is a *P_{lacl}q* promoter; preferably, the *P_{lacl}q* promoter comprises the sequence as shown in SEQ ID NO: 20.

17. The precursor plasmid of any one of claims 1-16, wherein the screened tag gene is an antibiotic resistant gene.

18. A host cell comprising:
1) a recombinase expression cassette expressing a recombinase; and
2) a second regulatory protein expression cassette expressing a second regulatory protein, wherein the second regulatory protein expression cassette comprises an expression regulatory sequence, and when bound to the expression regulatory sequence, the repressor protein inhibits the expression of the second regulatory protein.

19. The host cell of claim 18, wherein the recombinase is a Cre recombinase, a Flp recombinase, or a PhiC31 recombinase.

20. The host cell of claim 18 or 19, wherein the recombinase expression cassette is an inducible recombinase expression cassette, preferably an arabinose-inducible expression cassette.

21. The host cell of any one of claims 18-20, wherein the second regulatory protein is a product of *pir-116* coding gene; preferably, the *pir-116* coding gene comprises the sequence as shown in SEQ ID NO: 2.

22. The host cell of any one of claims 18-21, wherein the repressor protein is LacI protein.

23. The host cell of any one of claims 18-22, wherein the expression regulatory sequence comprises a *lacO* operator sequence; preferably, the *lacO* operator sequence comprises the sequence as shown in SEQ ID NO: 1.

24. The host cell of any one of claims 18-23, wherein the recombinase expression cassette and/or the second regulatory protein expression cassette are integrated in the genome of the host cell.

25. The host cell of any one of claims 18-24, which is *Escherichia coli.*

26. A method for preparing a screened-tag-gene-free plasmid, comprising:
I) preparing a precursor plasmid comprising
1) a conditioned replication initiation site having a plasmid replication initiation capacity that is dependent on regulatory proteins, wherein the conditioned replication initiation site has a first replication initiation state in the presence of a first regulatory protein and a second replication initiation state in the presence of a second regulatory protein, and has a stronger ability to initiate plasmid replication in the second replication initiation state than in the first replication initiation state;
2) a first regulatory protein expression cassette expressing the first regulatory protein;
3) a sequence encoding a repressor protein;
4) a screened tag gene;
5) a target gene, or a cloning site for inserting the target gene; and
6) paired recombination sites,
wherein the paired recombination sites enable the self-recombination of the precursor plasmid in the presence of a recombinase to form a subplasmid and a circular double-stranded DNA, wherein the subplasmid comprises the conditioned replication initiation site and the cloning site, or comprises the conditioned replication initiation site and the target gene, and the circular double-stranded DNA comprises the screened tag gene, the first regulatory protein expression cassette, and the sequence encoding a repressor protein;
II) introducing the precursor plasmid into a host cell, wherein the host cell comprises:
1) a recombinase expression cassette expressing the recombinase; and
2) a second regulatory protein expression cassette expressing the second regulatory protein,
wherein the second regulatory protein expression cassette comprises an expression regulatory sequence, and when bound to the expression regulatory sequence, the repressor protein inhibits the expression of the second regulatory protein;
III) screening for the host cell expressing the screened tag gene;
IV) culturing the host cell screened in step III), allowing the expression of the recombinase in the host cell, continuing to culture the host cell and screening for the host cell not expressing the screened tag gene; and
V) culturing the host cell screened in step IV) and extracting the plasmid.

27. The method of claim 26, wherein the presence of the repressor protein enables the inhibition of expression of the second regulatory protein in a host cell upon introduction of the precursor plasmid into the host cell.

28. The method of claim 26 or 27, wherein the sequence encoding a repressor protein is located in the first regulatory protein expression cassette such that the repressor protein is expressed in tandem with the first regulatory protein.

29. The method of any one of claims 26-28, wherein the conditioned replication initiation site is the *ori_{R6Kγ}* replication initiation site.

30. The method of any one of claims 26-29, wherein the first regulatory protein is a wild-type II protein and the second regulatory protein is a mutant of the wild-type II protein.

31. The method of any one of claims 26-30, wherein the second regulatory protein is a product of *pir-116* coding gene.

32. The method of any one of claims 26-31, wherein the sequences of the paired recombination sites are direct sequences.

33. The method of any one of claims 26-32, wherein the paired recombination sites are direct loxP sequences, and the recombinase is a Cre recombinase; the paired recombination sites are direct FRT sequences, and the recombinase is a Flp recombinase; or the paired recombination sites are direct attB/attP sequences, and the recombinase is a PhiC31 recombinase.

34. The method of any one of claims 26-33, wherein the paired recombination sites are direct lox71 and lox66 sequences.

35. The method of any one of claims 26-34, wherein the repressor protein is LacI protein and the expression regulatory sequence comprises a *lacO* operator sequence.

36. The method of any one of claims 26-35, wherein the screened tag gene is an antibiotic resistant gene.

37. The method of any one of claims 26-36, wherein the recombinase expression cassette is an inducible recombinase expression cassette, preferably an arabinose-inducible expression cassette.

38. The method of any one of claims 26-37, wherein the recombinase expression cassette and/or the second regulatory protein expression cassette are integrated in the genome of the host cell.

39. The method of any one of claims 26-38, wherein the host cell is *Escherichia coli.*

40. The method of any one of claims 26-39, wherein the allowing the expression of the recombinase in the host cell in step IV) is achieved by adding to the host cell an inducer corresponding to the inducible recombinase expression cassette.

41. A screened-tag-gene-free plasmid prepared by the method of any one of claims 26-40, the screened-tag-gene-free plasmid comprising the conditioned replication initiation site and the cloning site, or comprising the conditioned replication initiation site and the target gene.

42. A screened-tag-gene-free plasmid formed by self-recombination of the precursor plasmid of any one of claims 1-17 in the presence of a recombinase.

43. Use of the precursor plasmid of any one of claims 1-17 or the host cell of any one of claims 18-25 in the preparation of a screened-tag-gene-free plasmid.

44. A kit for the preparation of a screened-tag-gene-free plasmid, comprising the precursor plasmid of any one of claims 1-17 and/or the host cell of any one of claims 18-25.
